# EUROPEAN PATENT APPLICATION

(11) **EP 4 368 221 A1**
(43) Date of publication of application: **15.05.2024**
(21) Application number: 23208432.7
(22) Date of filing: 08.11.2023
(51) Int. Cl.: A61M 1/02

(54) **SYSTEM AND METHOD OF ALTERING BLOOD FLOWRATE DURING DONATION**

(30) Priority: 11.11.2022 US 202263424595 P
(71) Applicant: Fenwal, Inc., Lake Zurich, IL 60047 (US)
(72) Inventor: MADSEN, James G., Lake Zurich, 60047 (US); MIN, Kyungyoon, Lake Zurich, 60047 (US); GNIADEK, Thomas, Lake Zurich, 60047 (US)
(74) Representative: Maikowski & Ninnemann Patentanwälte Partnerschaft mbB

(57) **Abstract**

A system for altering blood flowrate during donation comprising a height adjustable blood collection assembly including: a base, a tower extending upward from the base, an actuator and a carrier; the actuator is coupled to the carrier and the tower, and the actuator vertically adjusts the carrier relative to the base; a receptacle being connected to the carrier and configured to receive a blood collection container; and a controller that interacts with at least the actuator.

## Description

### BACKGROUND

### Field of the Disclosure

The invention relates to blood collection systems. More particularly, the invention relates to devices used during whole blood collection, and the ability to alter the blood flowrate during blood donation.

### Description of Related Art

Blood donation procedures typically seek collection of Whole Blood (hereinafter "WB") from a donor who is sitting on a reclinable chair or lying on a reclinable bed. As such, these may be referred to collectively as bedside procedures. The current state of the art for blood donation is to utilize a blood collection device, such as CompGuard°, supplied by Fresenius Kabi KG, of Germany. The CompoGuard° device simultaneously controls the volume collected and subjects the collection container, such as a bag, to mixing. Upon determining that the proper amount of blood has been collected, the device will clamp the donation line and notify the phlebotomist or operator.

Current blood collection devices generally are designed to rest on a floor or table, or on a small travel case to a side of the donor's chair or bed. Flow rate from the donor to the collection bag is determined simply by the difference in height between the donor's arm and the collection bag resting on the collection device. Depending on the blood flowrate attempted to be achieved during the donation, the donor may experience hypotensive events, including adverse vasovagal reactions. The adverse reactions, although rare, may range from mild agitation, sweating, pallor, dizziness, and cold feeling to more severe, including loss of consciousness and vomiting. There is a need to be able to better alter and manage blood flowrates to help avoid vasovagal reactions during routine WB donations.

### SUMMARY

The present disclosure provides subject matter relating to a device and system for altering blood flowrate during WB donation. Control of the blood flowrate is based on use of a height adjustable device, which facilitates a reduction in the likelihood of adverse vasovagal reactions. The device may provide a slow initial flowrate, which may be increased throughout the donation procedure. By better controlling flowrate from the donor to the WB collection bag throughout the donation, hypotensive events may be reduced.

The height adjustable device may be provided in a new standalone blood collection device or may be incorporated into an existing blood collection system to provide an automated height adjustable platform. The device begins with the collection container in an initial raised or high position near the height of the donor's arm. This high position provides an initial slow donation flowrate which reduces the likelihood of a hypotensive event. The high position may be maintained until a threshold collection volume has been achieved, such as for a first 100 ml. Then, the device holding the collection container may slowly lower the collection container, such as toward a floor surface on which the device may be resting. The lowering of the collection container increases the donation flowrate, which permits a relatively quick donation time.

The advantage of the disclosed subject matter is an ability to alter the blood flowrate throughout the donation. Starting the donation with a slow flowrate, which may be increased gradually, whether on a continuous or stepped basis, may reduce the likelihood of adverse vasovagal reactions as the donor's blood supply is depleted gradually and potentially at an increasing rate. This is in contrast to existing blood donation devices which start and maintain a fast donor flowrate throughout the donation procedure. Thus, the device may be implemented within an automated system that will control the height of the collection container, and therefore, the flowrate in an effort to reduce the potential for vasovagal reactions that may be a reaction to an initial fast donor flowrate.

It will be appreciated that the height adjustable device for a WB collection system may be configured in a variety of constructions and for use in a variety of ways, some examples of which will be described herein.

The WB collection container may have any configuration and selected volume and, when combined into the system, must have at least one opening. Thus, the opening may be preexisting and connected to a flow path, or may be formed when the WB collection container is being connected to a flow path within the system. The flow path typically will be of a tubing construction. With the flow path connected to the collection container at one end, to undertake a donation procedure, the opposed end of the flow path must be connected to a needle. The needle must be appropriate for insertion into a patient.

In a first aspect, a system for altering blood flowrate during donation includes: a height adjustable blood collection assembly including: a base, a tower extending upward from the base, an actuator and a carrier; the actuator is coupled to the carrier and the tower, and the actuator vertically adjusts the carrier relative to the base; a receptacle being connected to the carrier and configured to receive a blood collection container; and a controller that interacts with the actuator

It will be appreciated that a system for altering blood flowrate during donation may be configured to include a height adjustable blood collection assembly, which may be constructed for use in advantageous, cost efficient and convenient ways.

### BRIEF DESCRIPTION OF THE DRAWINGS

FIG. 1 is a view of a first example of a donation system for altering blood flowrate during donation, in a bedside location.
FIGS. 2-4 are views of the first example donation system of FIG. 1 having the receptacle disposed in respective highest, intermediate and lowest positions.
FIG. 5 is view of the actuator and carrier coupled to the tower, and a receptacle connected to the carrier of the first example donation system of FIGS. 1-4.
FIG. 6 is a partial cross-sectional view of the components shown in FIG. 5.
FIG. 7 is a view of the actuator and tower shown in FIG. 5, including a portion of the carrier.
FIG. 8 is a view of the receptacle and a portion of the carrier shown in FIG. 5.
FIG. 9 is a view of an alternative actuator for use in the first example donation system of FIG. 1.
FIGS. 10 and 11 are views of a second example of a donation system for altering flowrate during donation and having the receptacle disposed in respective highest and lowest positions.
FIGS. 12 and 13 are partial cross-sectional views showing inner structures within the tower of the second example and in the respective highest and lowest positions corresponding to FIGS. 10 and 11.
FIGS. 14 and 15 are views of a third example of a donation system for altering flowrate during donation and having the receptacle disposed in respective highest and lowest positions.
FIGS. 16 and 17 are partial cross-sectional views showing inner structures within the tower of the third example and in the respective highest and lowest positions corresponding to FIGS. 14 and 15.
FIG. 18 is a flow chart providing steps of a method of operation of a donation system for altering blood flowrate during donation, which may be utilized with any of the example systems shown in FIGS. 1-17.

### DETAILED DESCRIPTION

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

It will be appreciated that a donation system for altering blood flowrate during donation may be applicable for efficient and convenient use with bedside blood donation systems, for the reduction of the likelihood of vasovagal reactions that may occur during donation procedures.

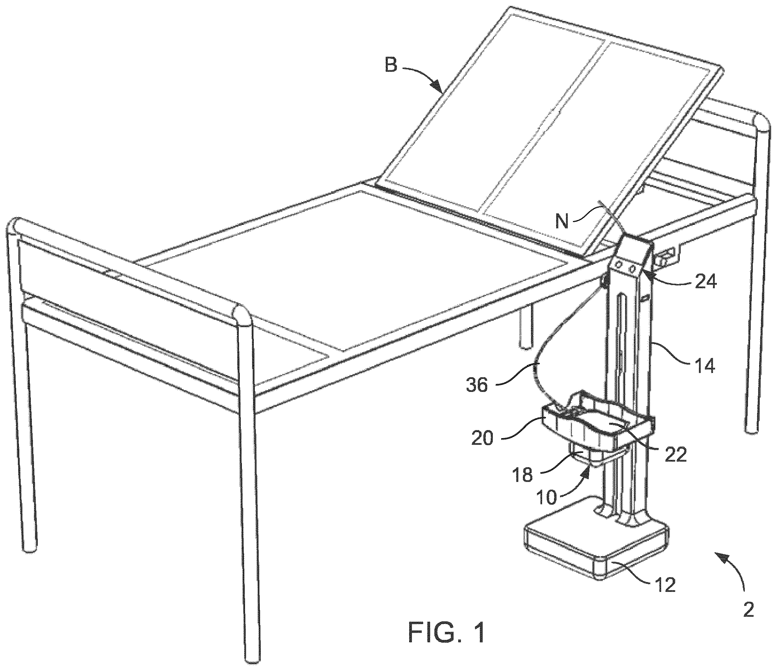

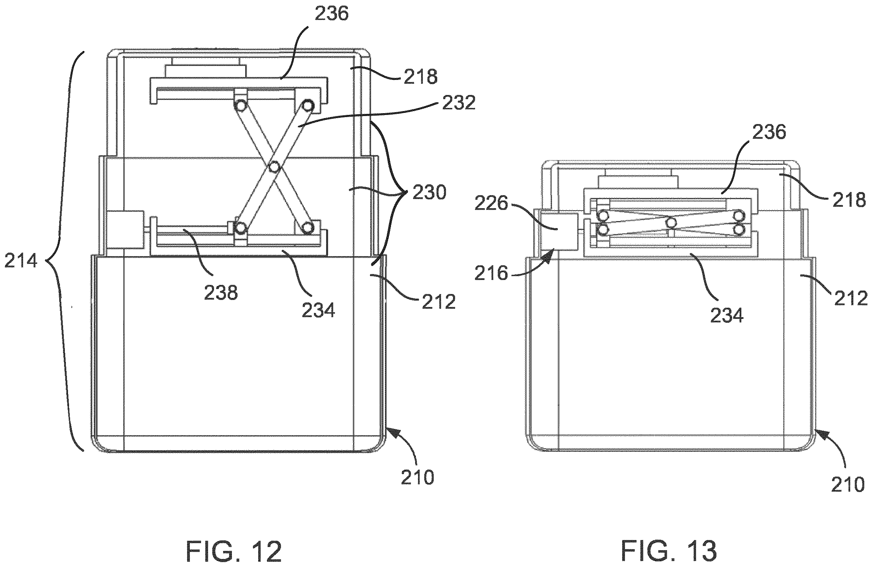

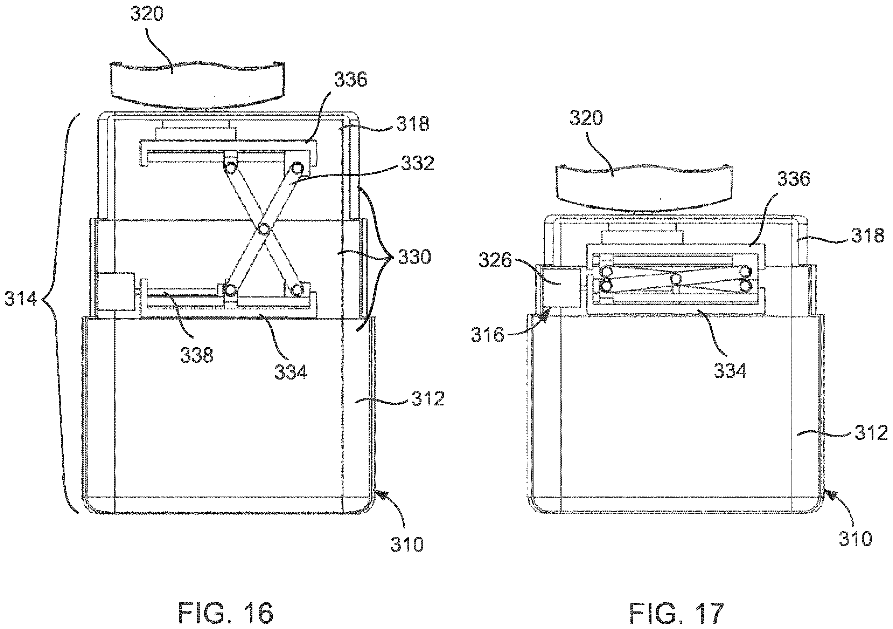

FIGS. 1-18 illustrate three example embodiments of such donation systems for altering blood flowrate during donation. Turning to FIGS. 1-8, the first example provides a system 2 for altering blood flowrate during donation comprising: a height adjustable blood collection assembly 10 including: a base 12, a tower 14 extending upward from the base 12, an actuator 16 and a carrier 18; the actuator 18 is coupled to the carrier 20 and the tower 16, and the actuator 18 vertically adjusts the carrier 18 relative to the base 12; a receptacle 20 being connected to the carrier 18 and configured to receive a blood collection container 22; and a controller 24 that interacts with at least the actuator 18. The system 2 is shown with the base 12 configured to rest on a horizontal surface, such as a floor surface. The system also is positioned bedside relative to what will be appreciated to be a donation chair or bed B on which a donor may rest during a WB donation procedure.

The actuator 16 includes a drive 26. The actuator 16 is shown as a linear actuator in the first example and the drive 26 provides rotatable movement. In this first example configuration, the tower 14 includes a column 30 extending upward from the base 12 and the carrier 18 translates vertically along the column 30. The actuator 16 further includes a vertically extending screw 28 located in the column 30. It will be appreciated that the drive 26 is connected to the base 12 and rotatably drives the screw 28 in the column 30.

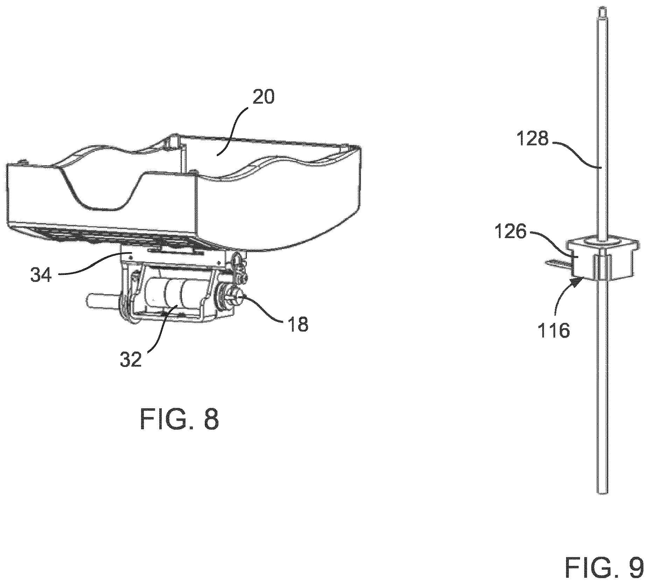

An alternative actuator 116 for the first example is shown in FIG. 9. The actuator 116 differs in that it includes a fixed screw 128 and the drive 126 further comprises a non-captive motor that rotates about the fixed screw 128. Thus, this alternative differs from the first example because the screw 128 is fixed in the column 30, the drive 126 is disposed above the base 12, the drive 126 is connected to the carrier 18 and translates relative to the fixed screw 128.

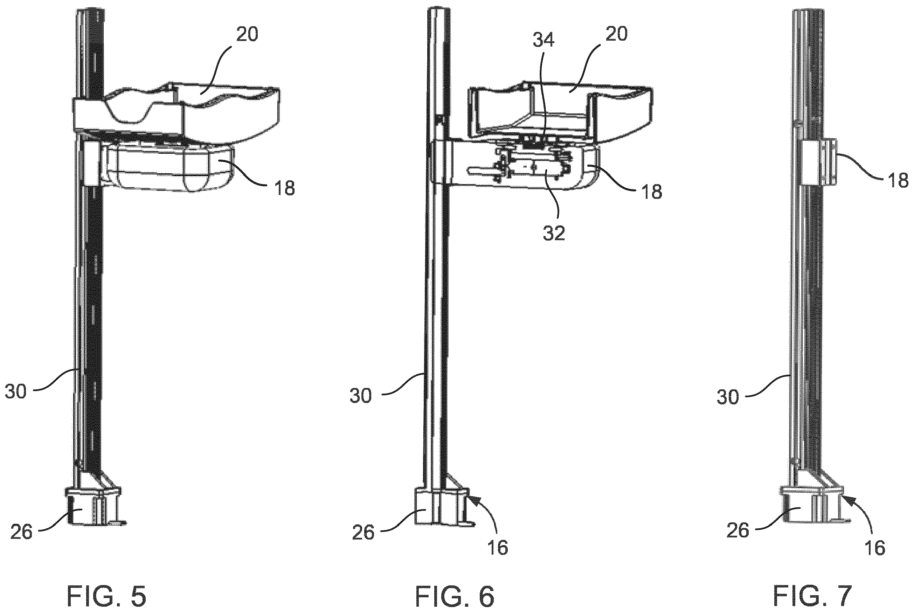

In the system 2, the receptacle 20 is movably connected to the carrier 18. It will be appreciated in FIGS. 6 and 8 that the carrier 18 may include a tilting mechanism 32 that provides tilting motion to the receptacle 20, for mixing of the contents within the blood collection container 22. In addition, the carrier 20 may further include a weight scale 34 operably connected to the controller 24 to weigh a blood collection container 22 received by the receptacle 20. During a blood collection procedure, the blood collection container 22 is connected to a flow path 36. It will be appreciated that a needle N will be connected to the flow path 36 at an end opposite the blood collection container 22, to provide fluid connection to the donor. The system 10 also may include at least one sensor 38 that monitors the flow path 36. An example of such a sensor may be an air detector. For managing operation of the system 2, it may further include at least one clamp 40 configured to engage the flow path 36. A clamp may be configured to be utilized in automated operation or to be applied manually by an operator.

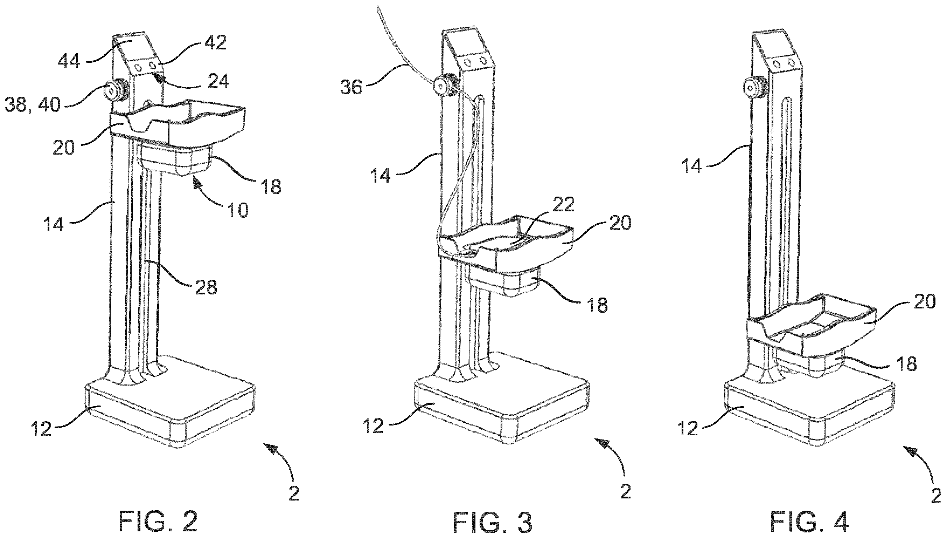

The system 2 preferably may operate in an automated manner or permit manual intervention to operate a donation procedure, and may include at least one input element 42, such as may be seen in FIG. 2. The at least one input element 42 is usable to enter a manual selection of a height adjustment of the carrier 18 and/or is usable to enter parameters relating to an automated donation procedure. Such other parameters may include, for example, the desired volume of blood to be donated, intervals of volume collected at which changes in the height of the height adjustment assembly should take place, as well as the respective heights associated with the respective volume intervals and/or other alternative suitable parameters. The at least one input element 42 will be coupled to the controller 24 to provide information used for the donation procedure. The controller 24 also may include a visual display 44, such as a readout or touch screen, as seen in FIG. 2.

The system 2 may be advantageously used in a donation procedure wherein the flow rate is increased during the procedure. For example, the donation procedure may be initiated with the carrier 20 and blood collection container 22 in a first position, which typically would be a highest of the positions to be used during the donation procedure, such as is shown in FIG. 2, until a first volume of blood has been collected. With the blood collection container 22 at a heigh relatively close to the height of the needle access site on the donor, the blood flow rate of the donation will be fairly low.

The donor's body will be permitted to adjust to the donation procedure during this time and once the first volume is achieved, the actuator 18 may move the carrier 18 and blood collection container 20 downward along the tower 14 via the drive 26 to locate the carrier 18 and blood collection container 22 in an intermediate height position, such as is shown in FIG. 3. This will increase the flow rate relative to the initial higher position shown in FIG. 2 and will quicken the pace of the procedure.

The second position shown in FIG. 3 may be held until a second volume of blood has been collected. With the donor's body having had the chance to adapt to and tolerate the increased flow rate relating to the second position, the actuator 18 may again be engaged to move the carrier 18 and blood collection container 20 further downward along the tower 16 via the drive 28. This will locate the carrier 18 and blood collection container 22 in a lowest position for the procedure, such as is shown in FIG. 4, inducing a still faster flow rate than when in either of the previous two relatively higher positions.

By utilizing the height adjustable blood collection assembly 10 of the system 2, and the three example positions, the system 2 may achieve a faster overall flow rate than may be achieved if the blood collection container 20 is to be held in a single position, wherein the height may present a compromise that appears to generate a relatively fast initial flow rate, but then does not increase during the donation procedure. While the present example illustrates use of three different heigh positions for the blood collection container 20, it will be appreciated that any number of different height positions may be utilized, the height may alternatively be changed continuously, and a procedure may include only downward movements of the carrier 18 and blood collection container 20, or may include later upward movement during the procedure to reduce or taper the blood collection flow rate if any adverse reactions are encountered and/or when approaching the conclusion of the procedure to avoid a more abrupt or higher magnitude drop in flow rate. It will be appreciated by one of ordinary skill in the art that these same advantages may be achieved with the other example systems provided herein.

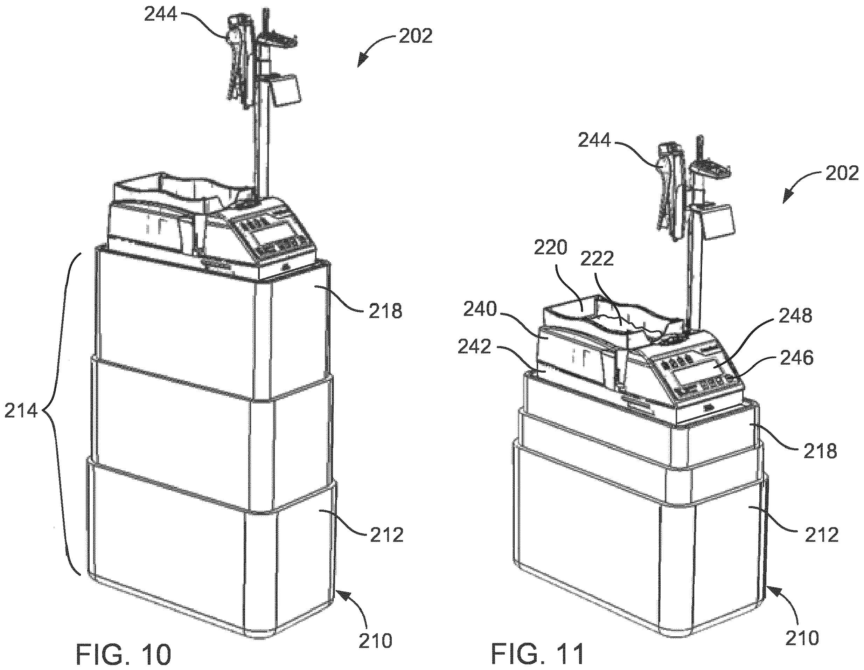

Turning to FIGS. 10-13, a second example system 202 for altering blood flowrate during donation is provided. The second example provides a system 202 for altering blood flowrate during donation comprising: a height adjustable blood collection assembly 210 including: a base 212, a tower 214 extending upward from the base 212, an actuator 216 and a carrier 218; the actuator 216 is coupled to the carrier 220 and the tower 214, and the actuator 216 vertically adjusts the carrier 218 relative to the base 212; a receptacle 220 being connected to the carrier 218 and configured to receive a blood collection container 222; and a controller 224 that interacts with at least the actuator 218. The system 202 is shown with the base 212 configured to rest on a horizontal surface, such as a surface of a floor or table. Thus, similar to the first example system 2, the second example system 202 may be positioned bedside for a WB donation procedure.

The actuator 216 includes a drive 226. The actuator 216 in this example is shown as a linear actuator and the drive 226 provides rotatable movement. In this example configuration, the tower 214 is telescopic and includes a plurality of telescoping riser elements 230. Among the plurality of telescoping riser elements 230 in the second example system 202 is the base 212, which is defined by a lowermost telescoping riser element and the carrier 218, which is defined by an uppermost telescoping riser element. The actuator 216 is located within the tower 214 and the actuator further comprises a scissor lift 232. The scissor lift 232 includes a lower portion 234 connected to the base 212 and an upper portion 236 connected to the carrier 218, and the scissor lift 232 moves the carrier 218 vertically relative to the base 212. The scissor lift 232 further includes a screw 238 and a drive 226 rotatably drives the screw 238.

In the second example system 202, the receptacle 220 is movably connected to the carrier 218. This connection includes a tilting mechanism 240 that provides tilting motion to the receptacle 220, for mixing of the contents within the blood collection container 222. The system 202 also is shown as including a weight scale 242 connected to the controller 224 to weigh a blood collection container 222 received by the receptacle 220. As with the first example, the blood collection container 222 would be connected to a flow path that provides fluid communication access to the donor, such as via a needle. The system 202 also may provide at least one sensor to monitor the flow path, similarly to the above discussion with respect to example sensors that may be used with the first example. To help control or seal off flow, the system 202 may include at least one clamp 244 configured to engage the flow path.

In addition, the system 202 may further include at least one input element 246 wherein the at least one input element 246 is usable to enter a manual selection of a heigh adjustment of the carrier 218 and/or is usable to enter parameters relating to a donation procedure. The controller 224 of the second example system 202 may further include a visual display 244, such as discussed above with respect to the first example system 2. The second example system 202 presents a configuration that include a combination of a height adjustable assembly 210 and the remainder of the system 202, which may otherwise be configures as a standalone donation processing system, which is resting upon the top of the height adjustable assembly 210.

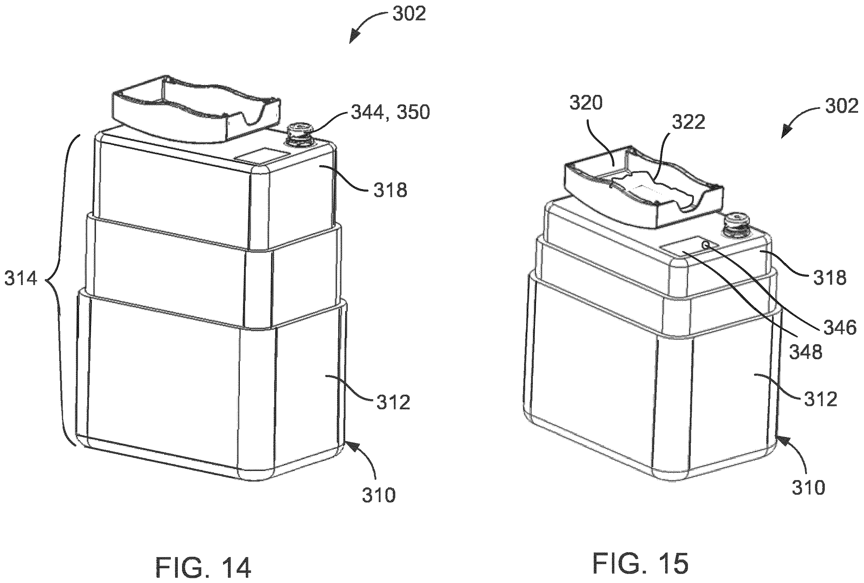

Now turning to FIGS. 14-17, a third example system 302 for altering blood flowrate during donation is provided. The third example provides a system 302 for altering blood flowrate during donation comprising: a height adjustable blood collection assembly 310 including: a base 312, a tower 314 extending upward from the base 312, an actuator 316 and a carrier 318; the actuator 316 is coupled to the carrier 320 and the tower 314, and the actuator 316 vertically adjusts the carrier 318 relative to the base 312; a receptacle 320 being connected to the carrier 318 and configured to receive a blood collection container 322; and a controller 324 that interacts with at least the actuator 318. The system 302 is shown with the base 312 configured to rest on a horizontal surface, such as a surface of a floor or table. Thus, similar to the first and second example systems 2, 202, the third example system 302 may be positioned bedside for a WB donation procedure.

The actuator 316 includes a drive 326. The actuator 316 in this example is similar to the actuator 216 of the second example and is shown as a linear actuator wherein the drive 326 provides rotatable movement. Similar to the second example, in the third example configuration, the tower 314 is telescopic and includes a plurality of telescoping riser elements 330. Among the plurality of telescoping riser elements 330 in the third example system 302 is the base 312, which is defined by a lowermost telescoping riser element and the carrier 318, which is defined by an uppermost telescoping riser element. The actuator 316 is located within the tower 314 and the actuator further comprises a scissor lift 332. The scissor lift 332 includes a lower portion 334 connected to the base 312 and an upper portion 336 connected to the carrier 318, and the scissor lift 332 moves the carrier 318 vertically relative to the base 312. The scissor lift 332 further includes a screw 338 and a drive 326 rotatably drives the screw 338.

In the third example system 302, the receptacle 320 is movably connected to the carrier 318. This connection includes a tilting mechanism 340 that provides tilting motion to the receptacle 320, for mixing of the contents within the blood collection container 322. The system 302 also is shown as including a weight scale 342 connected to the controller 324 to weigh a blood collection container 322 received by the receptacle 320. As with the first example, the blood collection container 322 would be connected to a flow path that provides fluid communication access to the donor, such as via a needle. The system 302 also may provide at least one sensor 350 to monitor the flow path, similarly to the above discussion with respect to example sensors that may be used with the first example. To help control or seal off flow, the system 302 may include at least one clamp 344 configured to engage the flow path.

In addition, the system 302 may further include at least one input element 346 wherein the at least one input element 346 is usable to enter a manual selection of a heigh adjustment of the carrier 318 and/or is usable to enter parameters relating to a donation procedure. The controller 324 of the third example system 302 may further include a visual display 344, such as discussed above with respect to the first and second example systems 2, 202. The third example system 302 presents a configuration that differs from that of the second example system 202 in that it includes similar height adjustable assembly 310, but then the remainder of the third example system 302 is configured to be more integrally constructed with the heigh adjustable assembly 310.

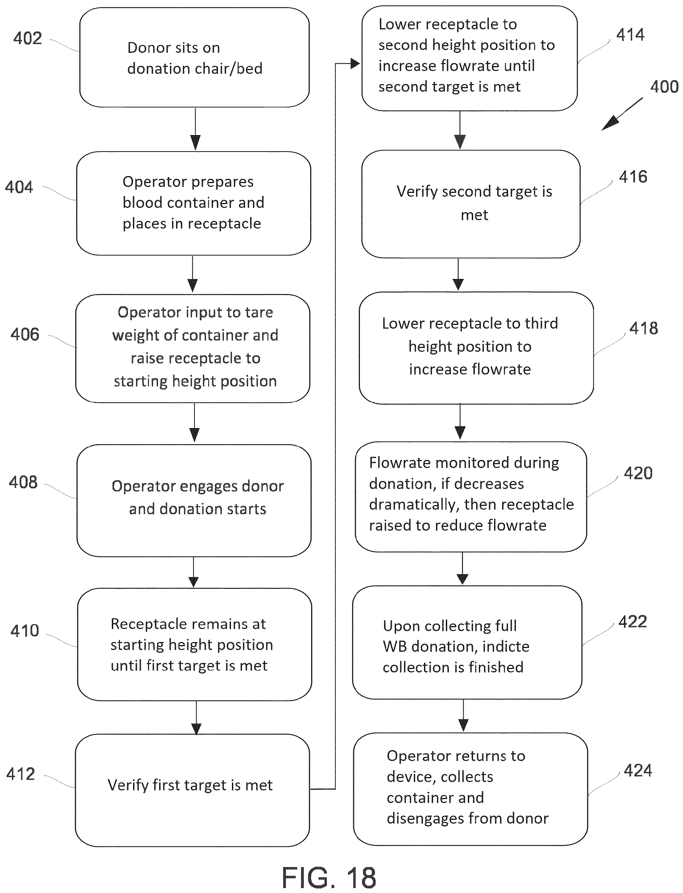

Turning to FIG. 18, a method 400 of performing a blood donation utilizing a system for altering blood flowrate during donation. The method may be achieved via use of the controller 24 and an automated program, or the operator could make all observations and adjustments manually, or intervene in an automated procedure, if for any reason such intervention were to become necessary. For purposes of this disclosure, the example method will be described with respect to the first example system 2.

In the initial step 402, the donor sits on the donation chair or bed B. Next, at step 404, the operator prepares a blood collection container 22 and places it in a height adjustable receptacle 20. At a step 406, the operator then may provide input to determine a tare weight of the blood collection container 22 and raises the receptacle to a starting height position, such as is shown in FIG. 2, which is likely to be close to the height of the fluid communication access with the donor, such as the height of an inserted needle. Next, at step 408, the operator engages the donor, by inserting a needle N connected to a flow path 36 at a first end, with the flow path 36 connected at a second end to the blood collection container 22. At step 410, the donation is started and the receptacle 20 remains at the starting height position until a first target volume of the collection is met. At step 412, the operator will verify that the first targe is met. The relatively slow initial flowrate permits the donor's body to adapt to and better tolerate the donation, reducing the likelihood of adverse reactions.

Next, at step 414, the receptacle is lowered to a second height position to increase flowrate to the blood collection container 22 until a second target volume of the collection is met. The second height, such as is shown in FIG. 3, is below the height of the needle engagement with the donor. At step 416, the operator will verify that the second target is met. The increased, second flowrate again permits the donor's body to adjust to the donation procedure with a reduced likelihood of adverse reactions.

Then, at step 418, the receptacle is lowered to a third height position to further increase flowrate to the blood collection container 22. The third height, such as is shown in FIG. 4, is below the first and second height positions. At step 420, the flowrate is monitored during donation and if it decreases dramatically, then the receptacle is raised to reduce the flowrate. At step 422, upon collecting the full WB donation, the system 2 indicates the collection is finished. The indication may be via a light or sound indicator. Finally, at step 424, the operator returns to the device, collects the blood collection container 22 and disengages the system 2 from the donor by removing the needle N. It will be appreciated that the donation procedure may include two or more height positions or include continuous height adjustment of the blood collection container supported by the receptacle 22 on the carrier 20. It will be appreciated that the graduated increases in flowrate, whether in two or more steps or on a continuous basis, is likely to help avoid vasovagal reactions during routine WB donations.

Accordingly, example systems and methods of altering blood flowrate during donation are disclosed and may be used to conduct blood donations with decreased risk of potentially problematic vasovagal reactions.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

### Other Aspects

Aspect 1. A system for altering blood flowrate during donation comprising
a height adjustable blood collection assembly including: a base, a tower extending upward from the base, an actuator and a carrier; the actuator is coupled to the carrier and the tower, and the actuator vertically adjusts the carrier relative to the base; a receptacle being connected to the carrier and configured to receive a blood collection container; and a controller that interacts with at least the actuator.

Aspect 2. The system of Aspect 1 wherein the base is configured to rest on a horizontal surface.

Aspect 3. The system of any of Aspects 1-2 wherein the actuator further comprises a drive.

Aspect 4. The system of Aspect 3 wherein the actuator is a linear actuator and the drive provides rotatable movement.

Aspect 5. The system of any of Aspects 1-4 wherein the actuator further comprises a screw and the drive rotates the screw.

Aspect 6. The system of any of Aspects 1-5 wherein the tower further comprises a column extending upward from the base and the carrier translates vertically along the column.

Aspect 7. The system of Aspect 6 wherein the actuator further comprises a vertically extending screw located in the column.

Aspect 8. The system of Aspect 7 wherein the drive is connected to the base and rotatably drives the screw in the column.

Aspect 9. The system of any of Aspects 1-4 wherein the actuator further comprises a fixed screw and the drive further comprises a non-captive motor that rotates about the fixed screw.

Aspect 10. The system of Aspect 9 wherein the screw is fixed in the column, the drive is disposed above the base, the drive is connected to the carrier and translates relative to the fixed screw.

Aspect 11. The system of Aspect 1 wherein the tower is telescopic.

Aspect 12. The system of Aspect 11 wherein the telescopic tower further comprises a plurality of telescoping riser elements.

Aspect 13. The system of Aspect 12 wherein among the plurality of telescoping riser elements the base is defined by a lowermost telescoping riser element and the carrier is defined by an uppermost telescoping riser element.

Aspect 14. The system of Aspect 13 wherein the actuator is located within the tower.

Aspect 15. The system of Aspect 14 wherein the actuator further comprises a scissor lift.

Aspect 16. The system of Aspect 15 wherein the scissor lift further comprises a lower portion connected to the base and an upper portion connected to the carrier, and the scissor lift moves the carrier vertically relative to the base.

Aspect 17. The system of Aspect 16 wherein the scissor lift further comprises a screw, and a drive rotatably drives the screw.

Aspect 18. The system of any of Aspects 1-4 wherein the receptacle is movably connected to the carrier.

Aspect 19. The system of Aspect 18 further comprising a tilting mechanism that provides tilting motion to the receptacle.

Aspect 20. The system of any of Aspects 1-4 further comprising a weight scale operably connected to the controller to weigh a blood collection container received by the receptacle.

Aspect 21. The system of Aspect 20 wherein the blood collection container is connected to a flow path.

Aspect 22. The system of Aspect 21 further comprising at least one sensor that monitors the flow path.

Aspect 23. The system of any of Aspects 1-4 further comprising at least one clamp configured to engage the flow path.

Aspect 24. The system of Aspect 1 further comprising at least one input element wherein the at least one input element is usable to enter a manual selection of a height adjustment of the carrier and/or is usable to enter parameters relating to a donation procedure

Aspect 25. The system of Aspect 1 wherein the controller further comprises a visual display.

Aspect 26. A method of performing a blood donation utilizing a system for altering blood flowrate during donation, comprising the steps of: locating a donor in a donation chair or bed; preparing and placing a blood collection container in a height adjustable receptacle; raising the receptacle to a starting height position; engaging the donor, by inserting a needle connected to a flow path at a first end, with the flow path connected at a second end to the blood collection container; starting the donation and having the receptacle remain at the starting height position until a first target volume of the collection is met; lowering the receptacle to a second height position to increase flowrate to the blood collection container until a second target volume of the collection is met; lowering the receptacle to a third height position to further increase flowrate to the blood collection container; monitoring the flowrate during donation and if the flowrate decreases significantly, raising the receptacle to reduce the flowrate; upon completing the donation, providing an indication that the collection is finished; having an operator collect the blood collection container and disengage the system from the donor by removing the needle.

Aspect 27. The method of Aspect 26 further comprising a step of establishing an initial tare weight of the blood collection container when initially placing the blood collection container in the receptacle.

Aspect 28. The method of Aspect 26 wherein raising the receptacle to a starting height position further comprises raising the receptacle to a height generally near the height of the needle insertion site on the donor.

## Claims

1. A system for altering blood flowrate during donation comprising:
a height adjustable blood collection assembly comprising:
a base, a tower extending upward from the base, an actuator and a carrier;
the actuator is coupled to the carrier and the tower, and the actuator vertically adjusts the carrier relative to the base;
a receptacle being connected to the carrier and configured to receive a blood collection container; and
a controller that interacts with at least the actuator.

2. The system of claim 1 wherein the actuator further comprises a drive.

3. The system of claim 2 wherein the actuator is a linear actuator comprising a screw and the drive provides rotatable movement relative to the screw.

4. The system of claim 1 wherein the tower further comprises a column extending upward from the base and the carrier translates vertically along the column.

5. The system of claim 4 wherein the actuator further comprises a vertically extending screw located in the column and the drive is connected to the base and rotatably drives the screw in the column.

6. The system of claim 4 wherein the actuator further comprises a fixed screw located in the column and the drive further comprises a non-captive motor disposed above the base and being connected to the carrier, wherein the drive rotates about the fixed screw causing the carrier to translate relative to the fixed screw.

7. The system of claim 1 wherein the tower is telescopic and comprises a plurality of telescoping riser elements.

8. The system of claim 7 wherein among the plurality of telescoping riser elements the base is defined by a lowermost telescoping riser element and the carrier is defined by an uppermost telescoping riser element.

9. The system of claim 1 wherein the actuator is located within the tower and comprises a scissor lift which further comprises a lower portion connected to the base and an upper portion connected to the carrier, and the scissor lift moves the carrier vertically relative to the base via a drive.

10. The system of claim 1 wherein the receptacle is movably connected to the carrier and comprises a tilting mechanism that provides tilting motion to the receptacle.

11. The system of claim 1 further comprising a weight scale operably connected to the controller to weigh a blood collection container received by the receptacle.

12. The system of claim 11 wherein the blood collection container is connected to a flow path.

13. The system of claim 12 further comprising at least one sensor that monitors the flow path.

14. The system of claim 12 further comprising at least one clamp configured to engage the flow path.

15. The system of claim 1 further comprising at least one input element wherein the at least one input element is usable to enter a manual selection of a height adjustment of the carrier and/or is usable to enter parameters relating to a donation procedure.
